Europäisches Patentamt

European Patent Office

Office européen des brevets

(11) Numéro de publication : **0 305 243 B1**

(12) **FASCICULE DE BREVET EUROPEEN**

(19)

(45) Date de publication du fascicule du brevet :
**11.03.92 Bulletin 92/11**

(21) Numéro de dépôt : **88401961.3**

(22) Date de dépôt : **28.07.88**

(51) Int. Cl.⁵ : **A61K 35/16,** A61L 25/00,
C07K 3/24

(54) **Concentré de protéines coagulables par la thrombine, son procédé d'obtention et son utilisation thérapeutique.**

(30) Priorité : **30.07.87 FR 8710798**

(43) Date de publication de la demande :
**01.03.89 Bulletin 89/09**

(45) Mention de la délivrance du brevet :
**11.03.92 Bulletin 92/11**

(84) Etats contractants désignés :
**AT BE CH DE ES FR GB GR IT LI LU NL SE**

(56) Documents cités :
**EP-A- 0 085 923**
**EP-A- 0 101 935**
**EP-A- 0 131 740**
**GB-A- 2 041 942**
**US-A- 4 156 681**
**US-A- 4 374 061**
**US-A- 4 377 572**
**US-A- 4 640 834**

(56) Documents cités :
**ARZNEIM.-FORSCH. DRUG RES., vol. 34(I), no.
3, 1984, pages 287-290; H. RONNEBERGER et
al.: "Untersuchungen mit einem Fibrin-Kleber
an Rattenhaut-Stanzwunden"**

(73) Titulaire : **CENTRE REGIONAL DE
TRANSFUSION SANGUINE DE LILLE
19-21 rue Camille Guérin
F-59012 Lille (FR)**

(72) Inventeur : **Burnouf Myriana
5, rue du Docteur Schaffner
59136 Wavrin (FR)**
Inventeur : **Burnouf Thierry
5 rue du Docteur Schaffner
59136 Wavrin (FR)**

(74) Mandataire : **Lhuillier, René et al
ARMENGAUD JEUNE CABINET LEPEUDRY
52, avenue Daumesnil
F-75012 Paris (FR)**

## Description

La présente invention porte sur un concentré de protéines coagulables par la thrombine, son procédé d'obtention à partir de plasma total et son utilisation à des fins thérapeutiques.

Ce type de concentré peut être utilisé pour obtenir par redissolution un fibrinogène injectable ou une colle biologique.

Ainsi qu'on le sait, les injections de fibrinogène permettent de traiter les états d'hypofibrinogémie ou d'afibrinogénie constitutionnelle, avec ou sans hémorragies, et également les syndromes de défibrination aiguë avec hémorragies graves, en association avec le traitement étiologique, l'héparinothérapie ou les anti-fibrinolytiques.

Les colles biologiques, d'autre part, permettent d'apporter une aide efficace lors de certains épisodes cliniques tels que les greffes de peau, les sutures nerveuses ou artérielles, les cicatrisations rapides ou toute utilisation où un effet hémostatique et/ou bactériostatique et/ou esthétique est recherché. En effet, le fibrinogène, constituant majeur d'un tel concentré, subit une dégradation enzymatique au contact avec la thrombine activée par des ions calcium. Après élimination des fibrinopeptides A et B les monomères de fibrine polymérisent alors spontanément et génèrent de la fibrine soluble. La présence de Facteur XIII dans ce type de produit contribue à stabiliser la fibrine par des liaisons covalentes en la rendant insoluble, c'est-à-dire résistante aux solvants, par exemple l'urée. La fibrine ainsi stabilisée, en plus de son rôle coagulant, est plus résistante à la fibrinolyse et à la traction mécanique.

Pour ces différentes utilisations thérapeutiques il est nécessaire de disposer d'un produit d'origine plasmatique présentant une bonne solubilité et stable dans les conditions de son utilisation. En ce qui concerne les colles biologiques il est outre nécessaire qu'elles présentent, après mise en contact avec la thrombine calcique, un haut pouvoir adhésif et une grande élasticité.

L'obtention de telles caractéristiques est directement liée à la nature du produit, et donc à son mode de purification à partir du plasma. Il est donc utile de disposer d'une technique de préparation facilement mise en oeuvre à l'échelle industrielle mais qui soit aussi suffisamment douce pour ne pas altérer les qualités biochimiques du produit pour l'utilisation recherchée par les cliniciens.

On connaît déjà, notamment d'après les brevets français Nos 2 448 900 et 2 448 901, des colles renfermant du fibrinogène et du Facteur XIII. Ces produits sont obtenus à partir de cryoprécipité de plasma par traitement avec une solution tampon contenant un inhibiteur-activateur du plasminogène ou inhibiteur de la plasmine, qui se retrouve présent dans la colle à l'état lyophilisé.

Ces produits présentent des caractéristiques intéressantes. Toutefois, ces produits sont obtenus selon des méthodes de production du fibrinogène assez complexes qui nécessitent, pour avoir un mélange final satisfaisant, l'adjonction exogène d'autres protéines plasmatiques comme le Facteur XIII. De plus, il faut adjoindre en cours de production des inhibiteurs, tels des inhibiteurs de protéases, d'origine animale comme l'aprotinine bovine.

Par ailleurs les concentrés de protéines connus, en particulier ceux utilisés à titre de colles, ne se solubilisent pas en milieu aqueux à température ambiante et peuvent même être difficiles à solubiliser à 37°C, obligeant à ajouter dans le flacon de lyophilisation du produit un barreau aimanté et à disposer d'un agitateur magnétique pour accélérer la remise en solution.

Il serait donc tout à fait bénéfique de disposer d'une méthode simple de production de colle biologique permettant d'obtenir, sans apport exogène de protéines, un mélange protéinique répondant aux qualités souhaitées pour un tel produit. En particulier, pour l'utilisation en tant que colle, le concentré obtenu devrait posséder un contenu satisfaisant de fibrinogène, de Facteur XIII et de fibronectine.

Le procédé en question doit aussi s'adapter aisément à des modifications visant à l'introduction d'une étape d'inactivation virale. Le produit en résultant devrait se solubiliser en moins de 10 minutes à la température d'utilisation (généralement 18 à 20°C), sans nécessité d'un outillage particulier. Il faudrait aussi que le produit soit stable plusieurs heures pour en faciliter l'utilisation et en garantir l'efficacité clinique.

La Demanderesse a ainsi mis au point un procédé simple qui permet de préparer un concentré de protéines coagulables par la thrombine contenant, par le simple fait du fractionnement mis en oeuvre, plus de 70 % de fibrinogène coagulable par rapport à l'ensemble des protéines présentes, et une quantité satisfaisante de Facteur XIII endogène, par une technique utilisant une fraction plasmatique disponible dans tous les centres de fractionnement.

L'invention concerne donc un concentré de protéines coagulables par la thrombine obtenu par précipitation et traitement à l'éthanol de plasma humain ou animal, qui contient avantageusement plus de 70 % en poids de fibrinogène coagulable par rapport aux protéines totales et du Facteur XIII endogène. Ce concentré, contrairement à ceux actuellement connus, se solubilise en milieu aqueux à température ambiante et ceci rapidement, c'est-à-dire en moins de 10 mn pour une teneur en protéines pouvant atteindre 150 g/l. Par ailleurs il reste stable après reconstitution pendant au moins 24 h, lorsqu'il est conservé à une température comprise par exemple entre 4 et 37°C.

Ce concentré contient avantageusement, par gramme de protéines, au moins 100 UI de Facteur XIII endogène.

Il contient en outre une quantité équilibrée de fibronectine, en particulier comprise entre 0,03 et 0,10 g/gramme de protéines.

Pour une utilisation en tant que fibrinogène injectable on formule et conditionne le concentré de façon à obtenir après reconstitution par resolubilisation une teneur totale en protéines de l'ordre de 17 à 20 g/l. Pour une utilisation à titre de colle biologique cette teneur sera comprise entre 100 et 120 g/l environ.

Le concentré selon l'invention est obtenu par un procédé comportant une étape de précipitation à l'éthanol dilué à un pH voisin de la neutralité et à basse température, d'un plasma total non soumis à la cryoprécipitation.

On ajuste les conditions de mise en oeuvre du procédé de façon à obtenir un précipité peu dénaturé, évitant ainsi les dégradations que subiraient les protéines si l'étape de précipitation se déroulait dans les conditions industrielles classiques de traitement du plasma. Ainsi, en dehors des précautions prises, notamment au niveau de la concentration en éthanol et de la température, on traite de préférence le plasma dans des récipients de petite taille (par exemple 10 litres) munis de barreaux aimantés. Le plasma est donc mis au contact avec de l'éthanol à faible concentration, par exemple de 8 à 12 %, pendant une durée pouvant atteindre plusieurs jours et en tout état de cause supérieure à 24 heures. Le surnageant est alors décanté et est disponible pour la préparation d'autres dérivés.

Le précipité obtenu après centrifugation est lavé à l'éthanol, toujours à basse température, c'est-à-dire entre 0 et 6°C et à un degré alcoolique à 8 à 12 %, de préférence 10 %, et l'ensemble est centrifugé.

Le précipité est alors remis en solution dans un tampon Tris/citrate, éventuellement concentré, filtré et de préférence lyophilisé avant traitement ultérieur. Eventuellement, il peut être traité directement, c'est-à-dire à l'état non lyophilisé.

Le traitement ultérieur réalisé pour obtenir un concentré de protéines selon l'invention peut être mis en oeuvre selon deux variantes d'exécution.

Selon une première variante on remet le lyophilisat ou la pâte fraîche correspondante en solution eau ou tampon Tris/citrate contenant avantageusement de la lysine et on traite la solution par de l'éthanol dilué à chaud.

Selon cette variante le lyophilisat de fibrinogène, ou la pâte fraîche correspondante, est donc remis en solution à un taux de protéines de l'ordre de 50 g/l et soumis à un traitement à l'éthanol à 8 à 12 %, à une température comprise entre 30 et 40°C, préférentiellement à 35°C et pour une durée de l'ordre d'une heure et demie. Cette étape, réalisée de préférence en présence de lysine, contribue à la bonne remise en solution de la colle biologique lyophilisée, tout en apportant une sécurité accrue vis-à-vis de l'inactivation d'éventuels virus pathogènes, dont le virus du SIDA.

La quantité de lysine utilisée permet d'avoir par exemple une concentration de l'ordre de 0,1 à 0,2 g-/gramme de protéines dans le produit prêt à l'emploi.

L'éthanol est éliminé par ultra-ou diafiltration contre un tampon contenant préférentiellement une quantité de lysine correspondant à la quantité souhaitée dans le produit prêt à l'emploi mais avantageusement dépourvu de citrate de façon à ce que la concentration de ce composé dans le produit final soit la plus faible possible, étant donné son action apparemment néfaste dans les phénomènes de coagulation. On utilise par exemple un tampon Tris/NaCl/lysine.

Le produit est alors filtré stérilement, réparti dans son flacon d'utilisation et lyophilisé.

Selon la deuxième variante d'exécution on soumet le lyophilisat, ou la pâte fraîche correspondante, après remise en solution, à une étape d'inactivation virale, par exemple à un traitement par solvant et détergent. Les résidus de ce traitement sont alors éliminés de préférence par une étape de précipitation à froid par de l'éthanol dilué.

Selon cette variante le lyophilisat de fibrinogène, ou la pâte fraîche correspondante, est donc remis en solution à un taux de protéines de l'ordre de 20 g/l. Il est alors soumis à un traitement d'inactivation virale par exemple à un traitement au solvant et détergent selon le brevet EP-A-0 131 740.

Cette étape, réalisée avantageusement à une température supérieure à 24°C et pour une durée supérieure à 6 h, apporte une sécurité accrue vis-à-vis de l'inactivation d'éventuels virus pathogènes, dont le virus du SIDA et les virus des hépatites. Elle est préférentiellement mise en oeuvre en présence de lysine du produit prêt à l'emploi de l'ordre de 0,1 à 0,2 g/gramme de protéines.

L'élimination des agents d'inactivation virale conjointement à l'augmentation de la pureté du concentré protéique est assurée par précipitation des protéines à basse température par l'éthanol à 8 à 12 %. Après centrifugation, on élimine en effet dans le surnageant les agents d'inactivation virale et des protéines contaminantes comme l'albumine. Si nécessaire, le précipité peut être mis en contact de nouveau avec la solution éthanolique afin d'assurer une meilleure élimination des agents d'inactivation virale.

Le précipité est remis en solution dans une solution tampon Tris-citrate-lysine puis ultrafiltré et diafiltré, avant filtration stérilisante et répartition. L'ultrafiltration vise à éliminer l'éthanol de même que le citrate mais permet de maintenir en teneur constante en lysine de 0,1 à 0,2 g par gramme de protéines.

Lorsque le concentré selon l'invention sert pour une colle biologique, la reconstitution de la colle avant emploi s'effectue par une solution aqueuse d'aprotinine à 10000 UIK/ml, la solution résultante étant mélangée à de la thrombine calcique à 500 UI/ml.

Le produit s'utilise soit sous forme liquide dispensée par un système de double aiguille (colle biologique reconstituée d'une part, et thrombine calcique d'autre part) soit sous forme sèche (utilisation en poudre) ou par un système de vaporisation.

Les exemples suivants illustrent l'invention.

## EXEMPLE 1

### A - PREPARATION DU CONCENTRE PROTEIQUE

Le plasma utilisé est obtenu par centrifugation de sang et congelé à -35°C dans les 6 heures suivant le prélèvement. Pour la préparation du concentré il est décongelé à 37°C. On le soumet ensuite à une précipitation à l'éthanol à 10 %, à pH 7,2, à un taux de protéines de 52 g/l, à une température de 4°C. Le mélange est agité durant 30 minutes puis laissé à décanter pour une durée minimale de 24 heures à 4°C.

Le surnageant éthanolique est éliminé par centrifugation et le précipité, riche en particulier en fibrinogène et en Facteur XIII, est récupéré. Celui-ci est soumis à un lavage poussé à l'aide d'une solution à 10 % d'éthanol prérefroidi à +4°C puis de nouveau centrifugé.

Le précipité est alors remis en solution par une solution tampon Tris/citrate, puis concentré à 15-20 g/l de protéines, filtré et éventuellement lyophilisé.

Le produit résultant est remis en suspension à 50 g/l de protéines par une solution de lysine correspondant à une teneur en lysine du produit final de 0,1 à 0,2 g/gramme de protéines, puis subit une deuxième traitement à l'éthanol à 10 % pour une heure et demie à 35°C.

Après diafiltration pour éliminer le citrate et l'éthanol et amener le taux de protéines à la valeur adéquate, par exemple 35 g/l, le concentré est filtré, réparti stérilement et lyophilisé dans le flacon final pour une remise en solution en 0,5, 1, 2 ou 5 ml, par exemple, lors de l'utilisation.

### B) ANALYSE BIOCHIMIQUE DU CONCENTRE DE PROTEINES

La composition en protéines du produit est la suivante (exprimée par gramme de protéines) :
Fibrinogène 0,70-0,75 g (fibrinogène coagulable)
Facteur XIII endogène 100-250 UI
Fibronectine 0,05 - 0,10 g.

### Exemple 2

### A) PREPARATION DU CONCENTRE PROTEIQUE

Le plasma utilisé est obtenu par centrifugation de sang et congelé à -35°C dans les 6 heures suivant le prélèvement. Pour la préparation du concentré, il est décongelé à 37°C. On le soumet ensuite à une précipitation à l'éthanol à 10 %, à pH 7,2, à un taux de protéines de 52 g/l, à une température de 4°C. Le mélange est agité durant 30 minutes puis laissé à décanter pour une durée minimale de 24 heures à 4°C.

Le surnageant éthanolique est éliminé par centrifugation et le précipité, riche en particulier en fibrinogène et en Facteur XIII, est récupéré. Celui-ci est soumis à un lavage poussé à l'aide d'une solution à 10 % d'éthanol prérefroidi à +4°C puis de nouveau centrifugé.

Le précipité est alors remis en solution par une solution tampon Tris/citrate, puis concentré à 15-20 g/l de protéines, filtré et éventuellement lyophilisé.

Le produit résultant est remis en suspension à environ 20 g/l de protéines par une solution de lysine correspondant à une teneur en lysine du produit final de 0,1 à 0,2 g/gramme de protéines, puis est soumis à un traitement par solvant et détergent à 0,3 % de TNBP et 1 % de Tween® 80 pendant une durée supérieure à 6 h et à une température supérieure à 24°C.

Le solution est alors soumise à une précipitation alcoolique à l'aide d'éthanol à 10 %, à 4°C, et laissée à décanter pour environ 12 h.

Après centrifugation et élimination du surnageant (qui peut renfermer des agents d'inactivation virale), le précipité est récupéré puis remis en solution par un tampon Tris/citrate renfermant de la lysine en quantité correspondant à 0,1-0,2 g de lysine par gramme de protéines.

Après diafiltration pour ajuster la force ionique, éliminer le citrate et l'éthanol (mais en conservant la lysine) et amener la teneur en protéines à une valeur adéquate, par exemple de 35 g/l environ, le concentré est filtré, réparti stérilement et lyophilisé dans le flacon final pour une remise en solution en 0,5, 1, 2 ou 5 ml, par exemple, lors de l'utilisation.

### B) ANALYSE BIOCHIMIQUE DU CONCENTRE PROTEIQUE

La composition en protéines du produit est la suivante (exprimée par gramme de protéines)
Fibrinogène 0,90-0,95 g (fibrinogène coagulable)
Facteur XIII endogène 150-300 UI
Fibronectine 0,03-0,06 g.

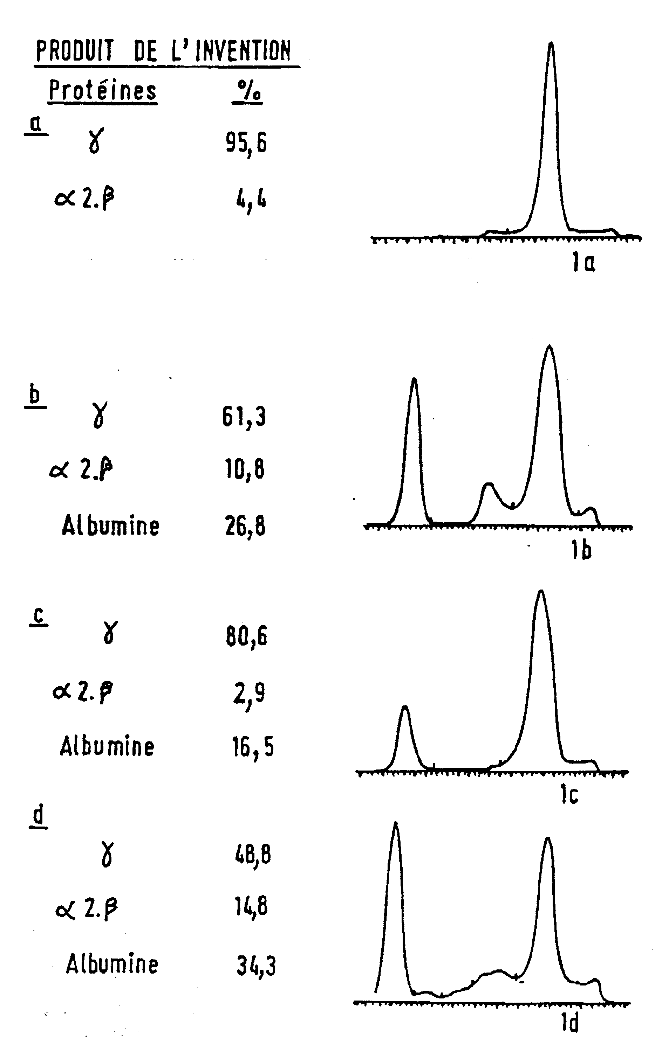

La figure unique annexée représente les résultats d'analyse par électrophorèse sur acétate de cellulose obtenus respectivement pour ce concentré (courbe 1a), et des concentrés du commerce utilisés en tant que colles biologiques ( courbes 1b à 1d).

La pureté en constituants de la zone γ (fibrinogène) largement supérieure du produit selon l'invention apparaît manifestement sur ces courbes.

Ainsi que mentionné plus haut ,le concentré selon l'invention présente d'excellentes caractéristiques de solubilisation et de stabilité.

En effet son temps de remise en solution est inférieur à 10 minutes, voire 5 minutes, tant à 20°C qu'à 37°C, sans appareillage particulier, par simple mouvement de rotation manuelle.

Par ailleurs on n'observe pas de déstabilisation du produit reconstitué et conservé à 4°C, 20°C ou 37°C pendant 24 h.

En ce qui concerne une colle biologique selon l'invention on constate qu'elle présente d'excellentes caractéristiques d'utilisation pour cette indication. En effet son pouvoir adhésif est supérieur à 100 g/cm$^2$ exprimé par test sur l'animal de collage de lambeaux de peau, valeur supérieure à celles obtenues sur d'autres colles préparées à partir de cryoprécipité. Ce pouvoir adhésif, se maintient à 90 % de sa valeur après reconstitution de la colle biologique et conservation 24 heures à 4°C ou à 20°C. Par ailleurs on ne constate pas d'exsudation lors du mélange du concentré protéique avec la thrombine calcique.

Les essais cliniques réalisés ont démontré la grande adaptabilité de cette colle aux contraintes de l'utilisation : besoin de remise en solution rapide, stabilité aux températures des blocs opératoires, possibilité d'attente d'utilisation.

Les indications d'une colle selon l'invention découlent de ses propriétés ; son pouvoir adhésif et hémostatique en font un adjuvant précieux des interventions chirurgicales au cours desquelles elle assure un gain de temps opératoire. En outre son pouvoir bactériostatique renforce et accélère la cicatrisation des plaies et des sutures.

Ses indications s'appliquent ainsi à des domaines très variés de la chirurgie :

– Chirurgie plastique et microchirurgie : greffe cutanée des brûlures, collages et lambeaux, lifting, blépharoplastie.

– Neurochirurgie : plastie et suture de dure-mère, hémostase d'exérèse tumorale, hémostase de sinus veineux.

– Chirurgie cardio-vasculaire : étanchéité des sutures des prothèses vasculaires, dissection aortique, anévrismes.

– Chirurgie générale et abdominale : collage de déchirures viscérales (splénique, rénale, hépatique...), ou de biopsie hépatique, anastomoses digestives, fistules, hémostase des cavités opératoires.

– Chirurgie osseuse : collage de corticale, suture du tendon, encollage de foyer ostéomyélitique.

– Stomatologie : hémostase de foyer d'extractions dentaires chez les malades à haut risque hémorragique (hémophiles).

– Oto-Rhino-Laryngologie : réparation de plaie tympanique, amygdaléctomie.

En ce qui concerne un fibrinogène injectable selon l'invention on constate qu'il présente également d'excellentes caractéristiques d'utilisation et que sa composition équilibrée en font un agent thérapeutique précieux pour le traitement des états d'hypo- ou d'afibrinogénémie et du syndrome de défibrination aiguë.

Pour le traitement des déficits constitutionnels, la dose usuelle est de 1 à 4 g suivant le poids du malade, en tenant compte que la demi-vie du fibrinogène est de trois à quatre jours et le taux plasmatique nécessaire pour avoir une hémostase suffisante est de l'ordre de 1g/litre.

Dans les états de défibrination aiguë les doses varient de 2 à 10 g suivant les circonstances.

Les produits thérapeutiques selon l'invention peuvent être préparés à partir de plasma humain ou animal et trouvent ainsi leur intérêt aussi bien en médecine humaine que vétérinaire.

**Revendications**

1. Procédé de préparation d'un concentré de protéines d'origine plasmatique coagulables par la thrombine, comprenant essentiellement du fibrinogène et du Facteur XIII, caractérisé en ce qu'il comprend les étapes suivantes :

a) une précipitation du plasma total non cryoprécipité par une solution aqueuse d'éthanol à 10 %, à 4°C et à pH 7,2, pendant 12 à 24 heures ;

b) l'élimination du surnageant et un lavage du précipité par de l'éthanol à 10 % à 4°C ;

c) une remise en solution du précipité en présence de lysine à une concentration correspondant à une teneur, dans le produit final, de 0,1 à 0,2 grammes par gramme de protéines ;

d) un traitement d'inactivation virale suivant une méthode classique par solvant-détergent ;

e) une seconde précipitation par de l'éthanol à 10 % ;

f) l'élimination du surnageant et une remise en solution du précipité en présence de lysine, comme à l'étape c), une diafiltration, une mise en flacons et une lyophilisation.

2. Procédé selon la revendication 1, caractérisé en ce que l'étape e) est effectuée à une température comprise entre 30 et 40°C pendant une durée d'environ 90 minutes.

3. Procédé selon la revendication 1, caractérisé en ce que l'étape e) est effectuée à une température de 4°C pendant une durée d'environ 12 heures.

4. Concentré de protéines obtenu par le procédé selon les revendications 1 et 2, caractérisé en ce qu'il contient, par gramme de protéines, de 0,70 à 0,75 g de fibrinogène, de 100 à 250 UI de Facteur XIII et de 0,05 à 0,10 g de fibronectine.

5. Concentré de protéines obtenu par le procédé selon les revendications 1 et 3, caractérisé en ce qu'il contient, par gramme de protéines, de 0,90 à 0,95 g de fibrinogène, de 100 à 250 UI de Facteur XIII et de 0,05 à 0,10 g de fibronectine.

6. Concentré de protéines selon la revendication 4 ou 5, caractérisé en ce qu'il se solubilise rapidement en milieu aqueux à température ambiante, jusqu'à une teneur en protéines de l'ordre de 150 g/l.

7. Concentré de protéines selon la revendication 4 ou 6, caractérisé en ce que sa teneur en protéines est ajustée entre 100 et 120 g/l pour le rendre utilisable comme colle biologique, par addition de thrombine calcique à 500 UI/ml.

8. Concentré de protéines selon la revendication 5 ou 6, caractérisé en ce que sa teneur en protéines est ajustée entre 17 et 20 g/l pour le rendre utilisable comme fibrinogène injectable, à usage thérapeutique.

**Claims**

1. Process for the preparation of a concentrate of thrombin-coagulable proteins derived from plasma comprising essentially fibrinogen and Factor XIII, characterised in that it comprises the following steps:
   a) precipitation of total plasma which has not been subjected to cryoprecipitation, by means of a 10 % aqueous ethanol solution, at 4°C and at pH 7.2, for a period of from 12 to 24 hours;
   b) removal of the supernatant and washing of the precipitate with 10 % ethanol at 4°C;
   c) resolubilisation of the precipitate in the presence of lysine at a concentration corresponding to a content in the end product of from 0.1 to 0.2 grammes per gramme of proteins;
   d) viral inactivation treatment according to a conventional solvent-detergent method;
   e) a second precipitation step by means of 10 % ethanol;
   f) removal of the supernatant and resolubilisation of the precipitate in the presence of lysine, as in step c), diafiltration, introduction into bottles, and lyophilisation.

2. Process according to claim 1, characterised in that step e) is carried out at a temperature of from 30 to 40°C for a period of approximately 90 minutes.

3. Process according to claim 1, characterised in that step e) is carried out at a temperature of 4°C for a period of approximately 12 hours.

4. Protein concentrate obtained by the process according to claims 1 and 2, characterised in that it contains, per gramme of proteins, from 0.70 to 0.75 g of fibrinogen, from 100 to 250 IU of Factor XIII and from 0.05 to 0.10 g of fibronectin.

5. Protein concentrate obtained by the process according to claims 1 and 3, characterised in that it contains, per gramme of proteins, from 0.90 to 0.95 g of fibrinogen, from 100 to 250 IU of Factor XIII and from 0.05 to 0.10 g of fibronectin.

6. Protein concentrate according to claim 4 or 5, characterised in that, up to a protein content of the order of 150 g/l, it dissolves rapidly in an aqueous medium at room temperature.

7. Protein concentrate according to claim 4 or 6, characterised in that its protein content is adjusted between 100 and 120 g/l to render it usable as a biological glue, by the addition of calcic thrombin at 500 IU/ml.

8. Protein concentrate according to claim 5 or 6, characterised in that its protein content is adjusted between 17 and 20 g/l to render it usable as injectable fibrinogen, for therapeutic use.

**Patentansprüche**

1. Verfahren zur Herstellung eines konzentrats von durch Thrombin koagulierbaren Plasma-Proteinen, die im wesentlichen Fibrinogen und den Faktor XIII enthalten, dadurch gekennzeichnet, daß es die folgenden Stufen umfaßt:
   (a) Präzipitation des nicht-kryopräzipitierten Gesamt-Plasmas mittels einer 10 %-igen wäßrigen Ethanollösung bei 4°C und bei pH 7,2 während eines Zeitraums von 12 bis 24 Stunden;
   (b) Eliminierung der überstehenden Flüssigkeit und Waschen des Niederschlags mit 10 %-igem Ethanol bei 4°C;
   (c) Wiederauflösung des Niederschlags in Gegenwart von Lysin in einer konzentration, die einem Gehalt in dem Endprodukt von 0,1 bis 0,2 g/g Proteine entspricht;
   (d) Durchführung einer Viren-Inaktivierungsbehandlung nach einer klassischen Methode mit einem Lösungsmittel-Detergens;
   (e) Durchführung einer zweiten Präzipitation mit 10 %-igem Ethanol;
   (f) Eliminierung der überstehenden Flüssigkeit und Wiederauflösung des Niederschlag-s in Gegenwart von Lysin wie in der Stufe (c), Durchführung einer Diafiltration, eines Abfüllens in Flaschen und einer Lyophilisierung.

2. Verfahren nach Anspruch 1, dadurch gekennzeichnet, daß die Stufe (e) bei einer Temperatur zwischen 30 und 40°C während einer Zeitdauer von etwa 90 Minuten durchgeführt wird.

3. Verfahren nach Anspruch 1, dadurch gekennzeichnet, daß die Stufe (e) bei einer Temperatur von 4°C während einer Zeitdauer von etwa 12 Stunden durchgeführt wird.

4. Protein-konzentrat, hergestellt nach dem Verfahren nach den Ansprüchen 1 und 2, dadurch gekennzeichnet, daß es pro g Proteine enthält 0,70 bis 0,75 g Fibrinogen, 100 bis 250 IU des Faktors XIII und 0,05 bis 0,10 g Fibronektin.

5. Proteinkonzentrat, hergestellt nach dem Verfahren nach den Ansprüchen 1 und 3, dadurch gekennzeichnet, daß es pro g Proteine enthält 0,90 bis 0,95 g Fibrinogen, 100 bis 250 IU des Faktors XIII und 0,05 bis 0,10 g Fibronektin.

6. Proteinkonzentrat nach Anspruch 4 oder 5, dadurch gekennzeichnet, daß es sich in einem wäßrigen Milieu bei Umgebungstemperatur schnell solubilisiert (auflöst) bis zu einem Gehalt an Proteinen in der Größenordnung von 150 g/l.

7. Proteinkonzentrat nach Anspruch 4 oder 6, dadurch gekennzeichnet, daß sein Gehalt an Proteinen auf einen Wert zwischen 100 und 120 g/l eingestellt wird, um es als biologischen klebstoff verwendbar zu machen, durch Zugabe von calciumhaltigem Thrombin in einer Menge von 500 IU/ml.

8. Proteinkonzentrat nach Anspruch 5 oder 6, dadurch gekennzeichnet, daß sein Gehalt an Proteinen auf einen Wert zwischen 17 und 20 g/l eingestellt wird, um es als injizierbares Fibrinogen verwendbar zu machen in der therapeutischen Verwendung.

## PRODUIT DE L'INVENTION

| | Protéines | % |
|---|---|---|
| a | $\gamma$ | 95,6 |
| | $\alpha$ 2.$\beta$ | 4,4 |
| b | $\gamma$ | 61,3 |
| | $\alpha$ 2.$\beta$ | 10,8 |
| | Albumine | 26,8 |
| c | $\gamma$ | 80,6 |
| | $\alpha$ 2.$\beta$ | 2,9 |
| | Albumine | 16,5 |
| d | $\gamma$ | 48,8 |
| | $\alpha$ 2.$\beta$ | 14,8 |
| | Albumine | 34,3 |

1a

1b

1c

1d